# EUROPÄISCHE PATENTSCHRIFT

(11) **EP 1 414 393 B1**
(45) Veröffentlichungstag und Bekanntmachung des Hinweises auf die Patenterteilung: **06.10.2010**
(21) Anmeldenummer: 02794739.9
(22) Anmeldetag: 01.08.2002
(51) Int. Cl.: A61K 8/49, A61Q 5/06, A61Q 5/10, D06P 3/14

(54) **MITTEL ZUM FÄRBEN VON KERATINHALTIGEN FASERN**
AGENT FOR COLORING KERATIN-CONTAINING FIBRES
PRODUITS DE COLORATION DE FIBRES CONTENANT DE LA KERATINE

(30) Priorität: 10.08.2001 DE 10139561
(43) Veröffentlichungstag der Anmeldung: 06.05.2004
(73) Patentinhaber: Henkel AG & Co. KGaA, 40589 Düsseldorf (DE)
(72) Erfinder: ROSE, David, 40723 Hilden (DE); MEINIGKE, Bernd, 51381 Leverkusen (DE); HÖFFKES, Horst, 40595 Düsseldorf (DE)
(86) Internationale Anmeldenummer: PCT/EP2002/008578
(87) Internationale Veröffentlichungsnummer: WO 2003/015733

(56) Entgegenhaltungen:
- EP-A- 1 025 834
- EP-A- 1 153 599
- EP-A- 1 166 757
- EP-A- 1 170 001
- WO-A-01/47477
- WO-A-95/01772
- WO-A-99/20234
- US-A- 3 749 714
- DATABASE WPI Section Ch, Week 198944 Derwent Publications Ltd., London, GB; Class B02, AN 1989-319875 XP002258687 -& JP 01 237452 A (SANPO KAGAKU KENKYUSHO KK), 21. September 1989 (1989-09-21)

## Beschreibung

Die Erfindung betrifft ein Mittel zum Färben von keratinhaltigen Fasern, insbesondere von menschlichen Haaren, das spezielle färbende Komponenten enthält, die Verwendung dieser speziellen Komponenten als färbende Komponente in Haarfärbemitteln sowie ein Verfahren zum Färben von keratinhaltigen Fasern, insbesondere von menschlichen Haaren.

Für das Färben von keratinhaltigen Fasern, beispielsweise Haaren, Wolle oder Pelzen, kommen im allgemeinen entweder direktziehende Farbstoffe oder Oxidationsfarbstoffe, die durch oxidative Kupplung einer oder mehrerer Entwicklerkomponenten untereinander oder mit einer oder mehreren Kupplerkomponenten entstehen, zur Anwendung. Kuppler- und Entwicklerkomponenten werden auch als Oxidationsfarbstoffvorprodukte bezeichnet.

Mit Oxidationsfarbstoffen lassen sich zwar intensive Färbungen mit guten Echtheitseigenschaften erzielen, die Entwicklung der Farbe geschieht jedoch i.a. unter dem Einfluß von Oxidationsmitteln wie beispielsweise H₂O₂, was in einigen Fällen Schädigungen der Faser zur Folge haben kann. Des weiteren können einige Oxidationsfarbstoffvorprodukte beziehungsweise bestimmte Mischungen von Oxidationsfarbstoffvorprodukten bisweilen bei Personen mit empfindlicher Haut sensibilisierend wirken. Direktziehende Farbstoffe werden unter schonenderen Bedingungen appliziert, ihr Nachteil liegt jedoch darin, daß die Färbungen häufig nur über unzureichende Echtheitseigenschaften verfügen.

Ziel der vorliegenden Erfindung ist es, Färbemittel für Keratinfasern, insbesondere menschliche Haare, bereitzustellen, die keins oder lediglich ein sehr geringes Sensibilisierungspotential aufweisen. Gleichzeitig sollen diese Färbemittel hinsichtlich ihrer Echtheitseigenschaften, der Farbtiefe und der Grauabdeckung qualitativ den üblichen Oxidationshaarfärbemitteln mindestens gleichwertig sein, ohne zwangsläufig auf die Verwendung von H₂O₂ angewiesen zu sein.

Es sind bereits eine Reihe von Mitteln zum Färben keratinhaltiger Fasern auf Basis kationischer direktziehender Farbstoffe bekannt, mit denen gute Färbeergebnisse erzielt werden können. So beschreibt WO 01/47477 A2 Mittel zum Färben keratinhaltiger Fasern, enthaltend spezielle färbende Komponenten, die eine Struktureinheit A-CH=B-CH=A' aufweisen. Aus WO 95/01772 A1 sind Färbemittel auf Basis bestimmter kationischer direktziehender Farbstoffe bekannt. Entsprechende kationische direktziehende Farbstoffe kommen auch gemäß WO 99/20234 A1 zum Einsatz, wobei die kationischen direktziehenden Farbstoffe in diesem Fall in Kombination mit selbstoxidierenden Farbstoffen eingesetzt werden. EP 1 025 834 A1 und EP 1 153 599 A2 offenbaren jeweils Haarfärbemittel, die eine Kombination zweier spezieller kationischer direktziehender Farbstoffe enthalten. Aus EP 1 170 001 A2 sind Haarfärbemittel bekannt, die einen ganz speziellen kationischen direktziehenden Farbstoff enthalten. Dennoch besteht beständig Bedarf an weiteren direktziehenden Farbstoffen.

Überraschend wurde nun gefunden, daß die in den Formeln
1) A4 und
2) A=B=A'
dargestellten Verbindungen sich auch in Abwesenheit von Oxidationsmitteln zum Färben von keratinhaltigen Fasern, insbesondere menschlichen Haaren, eignen. Die Ausfärbungen mit den erfindungsgemäßen Verbindungen verfügen über eine ausgezeichnete Brillianz und Farbtiefe und decken darüberhinaus einen breiten Farbbereich ab. Auf den Einsatz von Oxidationsmitteln soll jedoch nicht prinzipiell verzichtet werden.

Gegenstand der Erfindung ist ein Mittel zum Färben von keratinhaltigen Fasern, insbesondere menschlichen Haaren, enthaltend als färbende Komponente Verbindungen der Formel
1) A4 oder
2) A=B=A',
in denen =B= steht für eine durchgehende Doppelbindung zwischen den Gruppierungen A und A', oder =B= steht für die Gruppen B1 bis B3, 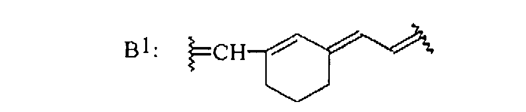 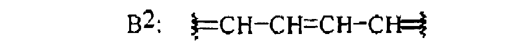 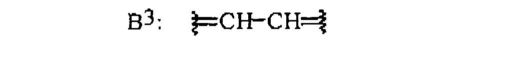 A ist ausgesucht aus den Gruppen A1 bis A3, 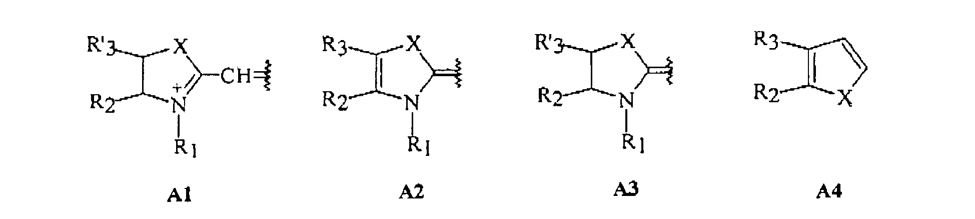 A' ist ausgerwählt aus den Gruppen A11 bis A14, 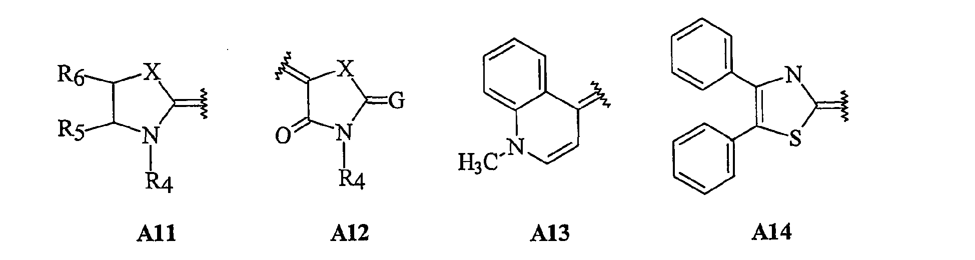 wobei
X steht für ein Schwefelatom, ein Sauerstoffatom, eine Gruppe NR', CR'R" oder eine Gruppe gemäß einer der Formeln X1 bis X4 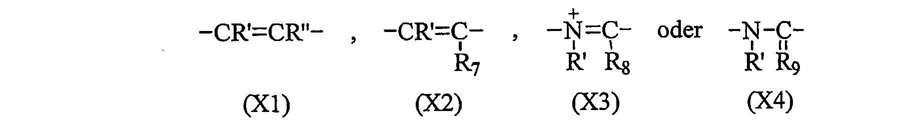 wobei R' und R" unabhängig voneinander stehen für Wasserstoff oder eine C₁-C₄-Alkylgruppe, die gegebenfalls mit einer SO₃-⁻Gruppe substituiert ist, R₇ steht für einen Rest gemäß der Formel (I), 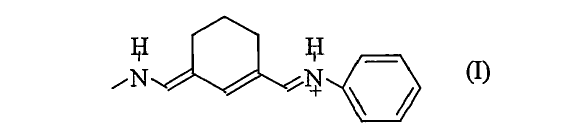 R₈ steht für einen Rest gemäß der Formel (II) 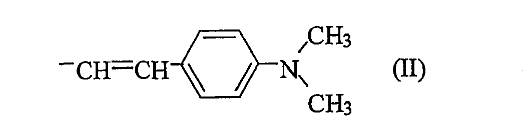 und R₉ steht für einen Rest gemäß Formel A12,
R₁ und R₄ stehen unabhängig voneinander für Wasserstoff, Amino, eine C₁-C₄-Alkylgruppe, die gegebenfalls mit einer oder mehreren Hydroxygruppe(n) oder einer Säure- oder Säureaniongruppe, insbesondere einer SO₃⁻-Gruppe substituiert sein kann, für eine C₁-C₄-Alkenylgruppe sowie für eine Formyl-, Acetyl-, Propionyl- und Buturylamidogruppe,
R₂ und R₃, R₂ und R'₃ sowie R₅ und R₆ können unabhängig voneinander stehen für Wasserstoff, eine C₁-C₄-Alkylgruppe, eine Formylgruppe- oder Acetyl- C₁-C₄-alkylestergruppe, eine Phenylgruppe oder sie können gemeinsam eine orthoankondensierte Cyclohexyl-, Benzyl- oder Naphthylgruppe bilden, die gegebenenfalls mit einem oder mehreren der folgenden Gruppen substituiert sein können: C₁-C₂-Alkyl, C₂-C₄-Mono- oder Polyhydroxyalkyl, Halogen, C₁-C₄-Alkoxy oder Formyl- oder Acetyl- C₁-C₄-alkylester; des weiteren kann R'₃ auch durch eine Doppelbindung an den Ring gebunden sein und für die Formel (III) stehen, 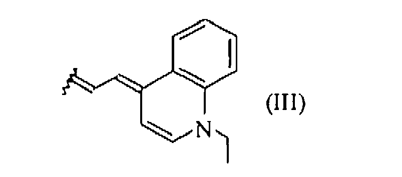 und G steht für ein Schwefelatom oder für eine Gruppe gemäß Formel (IV), 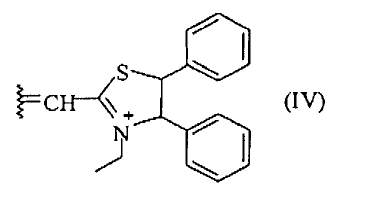 wobei diejenigen Formeln, die eine positive Ladung tragen weiterhin noch ein Gegenion enthalten, welches ausgesucht ist aus der Gruppe Halogenid, Ethylsulfat und 4-Methyl-benzol-sulfonat,
wobei als Verbindungen der Formel 1) und 2) eine oder mehrere der in Tabelle 1 genannten Verbindungen eingesetzt wird, wobei die in der Tabelle nicht definierten Substituenten für Wasserstoff stehen:

**Tabelle I**

| | **A** | **R₁** | **R₂/R₃ bzw- R₂/R'₃** | **B** | **A'** | **R₄** | **R₅//R₆** | **G** | **Gegenion** |
|---|---|---|---|---|---|---|---|---|---|
| **Verbindung 1** | A1 mit X=CR'R" Mit R' = R" = Methyl | Methyl | Benzol (ortho-ankondensiert) | B2 | A11 mit X = CR'R" mit R' = R" = Wassertoff | Wasserstoff, | Benzol (ortho-ankondensiert) | - | Chlorid |
| **Verbindung 2** | A1 mit X = S | Methyl | R₂ = R'₃ = Wasserstoff | B3 | A11 mit X = 0 | Wasserstoff | Naphthalin (ortho-ankondensiert) | - | 4-Methyl-benzolsulfonat |
| **Verbindung 3** | A1 mit X = S | Ethyl | Benzol (ortho-ankondensiert) | B1 | A11 mit X = S | Ethyl | Benzol (ortho-ankondensiert) | - | 4-Methyl-benzolsulfonat |
| **Verbindung 4** | A4 mit X= X2 mit R⁴ = Wasserstoff und R⁷ = Formel (I) | - | - | - | - | - | - | - | Chlorid |
| **Verbindung 5** | A1 mit X = X1 mit R' = R" = Wasserstoff | Methyl | Benzoyl (ortho-ankondensiert) | B3 | A11 mit X = S | Methyl | R₅ = R₆= Wasserstoff | - | 4-Methyl-benzolsulfonat |
| **Verbindung 6** | A1 mit X = S | Methyl | R₂ = R'₃ = Wasserstoff | 83 | A13 | - | - | - | 4-Methyl-benzolsulfonat |
| **Verbindung 7** | A1 mit X = S | Allyl | R₂ = Wasserstoff R'₃ = formel (III) | Durchgehende Doppelbindung | A14 | - | - | - | Ethylsulfat |
| **Verbindung 8** | A3 mit X = S | Methyl | R₂ = R'₃ = Wasserstoff | B3 | A12 mit X = 0 | Ethyl | - | S | - |
| **Verbindung 9** | A3 mit X = CR'R" Mit R' = R" = Wasserstoff | Methyl | R₂ = R'₃ = Wasserstoff | B3 | A12 mit X = NR' mit R' = Methyl | Methyl | - | S | - |
| **Verbindung 10** | A4 mit X = X3 mit R' = Ethyl R₈ = Formel (II) | - | - | - | - | - | - | - | Iodid |
| **Verbindung 11** | A2 mit X = S | Ethyl | R₂ = R₃ = Methyl | Durchgehende Doppelbindung | A12 mit X = S | Amino | - | S | - |
| **Verbindung 12** | A3 mit X = S | Ethyl | Benzol (ortho-ankondensiert) | B3 | A 12 mit X = S | Carboxymethyl | - | S | - |
| **Verbindung 13** | A3 mit X = S | Ethyl | R₂ = R'₃ = Phenyl | B3 | A12 mit X = S | Allyl | - | Formel (IV) | Ethylsulfat |
| **Verbindung 14** , | A3 mit X = CR'R" mit R' = R" = Wasserstoff | Methyl | R₂ = R'₃ = Wasserstoff | B3 | A12 mit X= 0 | Ethyl | - | S | - |
| **Verbindung 15** | A4 mit X = X₄ mit R' = Methyl und R₉ = A12; mit X = S, G = S, R₄ = Methyl | - | - | - | - | - | - | - | - |
| **Verbindung 16** | A4 mit X = X₄ mit R' = Methyl und R₉=A12; mit X=O, G = S, R₄ = Methyl | - | - | - | - | - | - | - | - |
| **Verbindung 17** | A2 mit X = S | Ethyl | R₂ = R₃ = Methyl | Durchgehende Doppelbindung | A12 mit X = S | Acetamido | - | S | - |
| **Verbindung 18** | A3 mit X = S | Methyl | R₂ = R'₃ = Wasserstoff | B3 | A12 mit X = NR' mit R' = Methyl | Wasserstoff | - | S | - |
| **Verbindung 19** | A3 mit X = 0 | Carboxy-ethyl | Benzol (ortho-ankondensiert) | B3 | A 12 mit X= NR' mit R' = Propyl | 2-Hydroxyethyl | - | S | - |
| **Verbindung 20** | A3 mit X = S | Methyl | R₂ = R'₃ = Wasserstoff | B3 | A12 mit X = S | Alkyl | - | S | - |
| **Verbindung 21** | A3 mit X = CR'R" mit R' = R" = Wasserstoff | Methyl | R₁=R'₃= Wasserstoff | B3 | A12 mit X = S | Allyl | - | S | - |
| **Verbindung 22** | A2 mit X = S | Ethyl | R₂ = R₃ = Methyl | Durchgehende Doppelbindung | A12 mit X = S | Allyl | - | S | - |
| **Verbindung 23** | A2 mit X = S | Ethyl | R₂ = Wasserstoff R₃ = Phenyl | Durchgehende Doppelbindung | A 12 mit X = S | Allyl | - | S | - |
| **Verbindung 24** | A2 mit X = S | Methyl | R₂ = Ethylcarboxymethyl R₃ = Wasserstoff | Durchgehende Doppelbindung | A 12 mit X = S | Allyl | - | S | - |
| **Verbindung 25** | A3 mit X= 0 | Carboxy-ethyl | Benzol (ortho-ankondensiert) | B3 | A12 mit X = NR' mit R' = Methyl | Methyl | - | S | - |
| **Verbindung 26** | A3 mitX=NR' mit R' = Ethyl | Ethyl | Ethylbenzoat (arom. Substituent nicht benachbart zur Kondensationsstelle) | B3 | A12 mit X=O | Ethyl | - | S | - |
| **Verbindung 27** | A3 mit X= NR' mit R' = Ethyl | Ethyl | Ethylbenzoat (arom. Substituent nicht benachbart zur Kondensationsstelle) | B3 | A12 mit X = S | Ethyl | - | S | - |

Unter keratinhaltigen Fasern sind Wolle, Pelze, Federn und insbesondere menschliche Haare zu verstehen. Die erfindungsgemäßen Färbemittel können prinzipiell aber auch zum Färben anderer Naturfasern, wie beispielsweise Baumwolle, Jute, Sisal, Leinen oder Seide, modifizierter Naturfasern, wie beispielsweise Regeneratcellulose, Nitro-, Alkyl- oder Hydroxyalkyl- oder Acetylcellulose und synthetischer Fasern, wie beispielsweise Polyamid-, Polyacrylnitril-, Polyurethan- und Polyesterfasern verwendet werden.

Beispiele für die als Substituenten in den erfindungsgemäßen Verbindungen genannten, C₁- bis C₄-Alkylgruppen sind die Gruppen Methyl, Ethyl, Propyl, Isopropyl und Butyl. Ethyl und Methyl sind bevorzugte Alkylgruppen. Erfindungsgemäß bevorzugte C₁- bis C₄-Alkoxyradikale sind beispielsweise eine Methoxy- oder eine Ethoxygruppe. Weiterhin können als bevorzugte Beispiele für eine C₁- bis C₄-Hydroxyalkylgruppe eine Hydroxymethyl-, eine 2-Hydroxyethyl-, eine 3-Hydroxypropyl- oder eine 4-Hydroxybutylgruppe genannt werden. Eine 2-Hydroxyethylgruppe ist besonders bevorzugt. Beispiele für Halogenatome sind erfindungsgemäß F-, Cl- oder Br-Atome, Cl-Atome sind ganz besonders bevorzugt. Beispiele für Formyl- oder Acetyl-C₁-C₄-Alkylester sind Essigsäure- oder Ameisensäuremethyl-, ethyl- propyl-, und butylester, besonders bevorzugt sind Ameisensäure- und Essigsäureethylester, Die weiteren verwendeten Begriffe leiten sich erfindungsgemäß von den hier gegebenen Definitionen ab,

Die Verbindungen der Formel 1) und 2) sind zum Teil literaturbekannt, im Handel erhältlich oder nach bekannten Syntheseverfahren herstellbar; ihre Verwendung in Haarfärbemitteln zum Färben von keratinhaltigen Fasern ist bislang nicht bekannt.

Die voranstehend genannten Verbindungen der Formel 1) und 2) werden vorzugsweise in den erfmdungsgemäßen Mitteln jeweils in einer Menge von 0,03 bis 30 mmol, insbesondere von 1 bis 10 mmol, bezogen auf 100 g des gesamten Färbemittels, verwendet.

Die erfindungsgemäß eingesetzten Verbindungen mit der Formel 1) und 2) können als direktziehende Färbemittel oder in Gegenwart von üblichen Oxidationsfarbstoffvorprodukten, wie üblichen Kuppler- und Entwicklerkomponenten eingesetzt werden.

Als Entwicklerkomponenten werden üblicherweise primäre aromatische Amine mit einer weiteren, in para- oder ortho-Position befindlichen freien oder substituierten Hydroxy- oder Aminogruppe, Diaminopyridinderivate, heterocyclische Hydrazone, 4-Aminopyrazolon-derivate sowie 2,4,5,6-Tetraaminopyridin und dessen Derivate eingesetzt.

Spezielle Vertreter sind beispielsweise p-Phenylendiamin, p-Toluylendiamin, 2,4,5,6-Tetraaminopyridin, p-Aminophenol, N,N-Bis-(2`-hydroxyethyl)-p-phenylendiamin, 2-(2',5-Diaminophenyl)-ethanol, 2-(2',5'-Diaminophenoxy)-ethanol, 1-Phenyl-3-carboxyamido-4-amino-pyrazolon-5, 4-Amino-3-methylphenol, 2-Aminomethyl-4-aminophenol, 2-Hydroxy-methyl-4-aminophenol, 2-Hydroxy-4,5,6-triaminopyridin, 2,4-Dihydroxy-5,6-diamino-pyrimidin und 2,5,6-Triamino-4-hydroxypyrimidin.

Als Kupplerkomponenten werden in der Regel m-Phenylendiaminderivate, Naphthole, Resorcin und Resorcinderivate, Pyrazolone, m-Aminophenole und substituierte Pyridinderivate verwendet.

Als Kupplersubstanzen eignen sich insbesondere α-Naphthol, 1,5-, 2,7- und 1,7-Dihydroxynaphthalin, 5-Amino-2-methylphenol, m-Aminophenol, Resorcin, Resorcinmono-methylether, m-Phenylendiamin, 2,4-Diaminophenoxyethanol, 2-Amino-4-(2'-hydroxyethyl-amino)-anisol, 1-Phenyl-3-methyl-pyrazolon-5, 2,4-Dichlor-3-aminophenol, 1,3-Bis(2',4'-diaminophenoxy)-propan, 2-Chlorresorcin, 4-Chlorresorcin, 2-Chlor-6-methyl-3-amino-phenol, 2-Methylresorcin, 5-Methylresorcin, 3-Amino-6-methoxy-2-methylaminopyridin und 3,5-Diamino-2,6-dimethoxypyridin.

Bezüglich weiterer üblicher Farbstoffkomponenten wird ausdrücklich auf die Reihe "Dermatology", herausgegeben von Ch. Culnan, H. Maibach, Verlag Marcel Dekker Inc., New York, Basel, 1986, Bd. 7, Ch. Zviak, The Science of Hair Care, Kap. 7, Seiten 248 - 250 (Direktziehende Farbstoffe), und Kap. 8, Seiten 264 - 267 (Oxidationsfarbstoffe), sowie das "Europäische Inventar der Kosmetikrohstoffe", 1996, herausgegeben von der Europäischen Komission, erhältlich in Diskettenform vom Bundesverband der deutschen Industrie- und Handelsunternehmen für Arzneimittel, Reformwaren und Körperpflegemittel, Bezug genommen.

Färbemittel, die als einzige färbende Komponente die erfindungsgemäßen Verbindungen enthalten, werden bevorzugt für Färbungen von gelb, über orange und rot bis hin zu türkis/violett eingesetzt.

Zur Erlangung weiterer und intensiverer Ausfärbungen können die erfindungsgemäßen Mittel zusätzlich Farbverstärker enthalten. Die Farbverstärker sind vorzugsweise ausgewählt aus der Gruppe bestehend aus Piperidin, Piperidin-2-carbonsäure, Piperidin-3-carbonsäure, Piperidin-4-carbonsäure, Pyridin, 2-Hydroxypyridin, 3-Hydroxypyridin, 4-Hydroxypyridin, Imidazol, 1-Methylimidazol, Histidin, Pyrrolidin, Prolin, Pyrrolidon, Pyrrolidon-5-carbonsäure, Pyrazol, 1,2,4-Triazol, Piperazin, deren Derivaten sowie deren physiologisch verträglichen Salzen.

Die voranstehend genannten Farbverstärker können in einer Menge von jeweils 0,03 bis 65, insbesondere 1 bis 40 mmol, jeweils bezogen auf 100 g des gesamten Färbemittels, eingesetzt werden.

In allen Färbemitteln können auch mehrere verschiedene Verbindungen mit der Formel 1) oder 2) zum Einsatz kommen; ebenso können auch mehrere verschiedene Oxidationsfarbstoffvorprodukte und/oder Farbverstärker gemeinsam verwendet werden.

Auf die Anwesenheit von Oxidationsmitteln, wie beispielsweise H₂O₂, kann dabei verzichtet werden. Es kann jedoch unter Umständen wünschenswert sein, den erfindungsgemäßen Mitteln zur Erzielung der Nuancen, die heller als die zu färbende keratinhaltige Faser sind, Wasserstoffperoxid oder Gemische von Wasserstoffperoxid und Peroxysalzen, beispielsweise Peroxydisulfit, als Oxidationsmittel zuzusetzen. Wasserstoffperoxid ist ein für menschliches Haar bevorzugtes Oxidationsmittel und wird in der Regel in einer Menge von 0,01 bis 6 Gew.-%, bezogen auf die Anwendungslösung, eingesetzt.

In einer bevorzugten Ausführungsform enthalten die erfindungsgemäßen Färbemittel zur weiteren Modifizierung der Farbnuancen neben den erfindungsgemäß enthaltenen Verbindungen zusätzlich übliche direktziehende Farbstoffe, zum Beispiel aus der Gruppe der Nitrophenylendiamine, Nitroaminophenole, Anthrachinone oder Indophenole, wie beispielsweise die unter den internationalen Bezeichnungen bzw. Handelsnamen HC Yellow 2, HC Yellow 4, HC Yellow 6, Basic Yellow 57, Disperse Orange 3, HC Red 3, HC Red BN, Basic Red 76, HC Blue 2, Disperse Blue 3, Basic Blue 99, HC Violet 1, Disperse Violet 1, Disperse Violet 4, Disperse Black 9, Basic Brown 16 und Basic Brown 17 bekannten Verbindungen sowie Pikraminsäure 2-Amino-6-chloro-4-nitrophenol, 4-Amino-2-nitrodiphenylamin-2'-carbonsäure, 6-Nitro-1,2,3,4-tetrahydrochinoxalin, 4-N-Ethyl-1,4-bis(2'-hydroxyethylamino)-2-nitrobenzol-hydrochlorid und 1-Methyl-3-nitro-4-(2'-hydroxyethyl)-aminobenzol. Die erfindungsgemäßen Mittel gemäß dieser Ausführungsform enthalten die direktziehenden Farbstoffe bevorzugt in einer Menge von 0,01 bis 20 Gew.-%, bezogen auf das gesamte Färbemittel.

Weiterhin können die erfindungsgemäßen Zubereitungen auch in der Natur vorkommende Farbstoffe wie beispielsweise Henna rot, Henna neutral, Henna schwarz, Kamillenblüte, Sandelholz, schwarzer Tee, Faulbaumrinde, Salbei, Blauholz, Krappwurzel, Catechu, Sedre und Alkannawurzel enthalten.

Es ist nicht erforderlich, daß die erfindungsgemäß enthaltenen Verbindungen der Formel 1) und 2) die fakultativ enthaltenen Oxidationsfarbstoffvorprodukte, Farbverstärker und direktziehenden Farbstoffe jeweils einheitliche Verbindungen darstellen. Vielmehr können in den erfindungsgemäßen Färbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, beispielsweise toxikologischen, ausgeschlossen werden müssen.

Die erfindungsgemäßen Färbemittel ergeben bereits bei physiologisch verträglichen Temperaturen von unter 45°C intensive Färbungen. Sie eignen sich deshalb besonders zum Färben von menschlichen Haaren. Zur Anwendung auf dem menschlichen Haar können die Färbemittel üblicherweise in einen wasserhaltigen kosmetischen Träger eingearbeitet werden. Geeignete wasserhaltige kosmetische Träger sind zum Beispiel Cremes, Emulsionen, Gele oder auch tensidhaltige schäumende Lösungen wie zum Beispiel Shampoos oder andere Zubereitungen, die für die Anwendung auf den keratinhaltigen Fasern geeignet sind. Falls erforderlich, ist es auch möglich, die Färbemittel in wasserfreie Träger einzuarbeiten.

Weiterhin können die erfindungsgemäßen Färbemittel alle in solchen Zubereitungen bekannten Wirk-, Zusatz- und Hilfsstoffe enthalten. In vielen Fällen enthalten die Färbemittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie beispielsweise eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ -CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Citronensäure mit Alkoholen, die Anlagerungsprodukte von etwa 2 bis 15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12

Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Als zwitterionische Tenside werden solche oberflächenaktiven Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammonium-glycinate, beispielsweise das Kokosacylaminopropyldimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der CTFA-Bezeichnung Cocamidopropyl Betaine bekannte Fettsäureamid-Derivat.

Unter ampholytischen Tensiden werden solche oberflächenaktiven Verbindungen verstanden, die außer einer C₈₋₁₈-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Nichtionische Tenside enthalten als hydrophile Gruppe beispielsweise eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂₋₂₂-Fettsäuremono- und -diester von Anlagerungsprodukten von 1 bis 30 Mol Ethylenoxid an Glycerin,
- C₈₋₂₂-Alkylmono- und -oligoglycoside und deren ethoxylierte Analoga,
- Anlagerungsprodukte von 5 bis 60 Mol Ethylenoxid an Rizinusöl und gehärtetes Rizinusöl,
- Anlagerungeprodukte von Ethylenoxid an Sorbitanfettsäureester
- Anlagerungsprodukte von Ethylenoxid an Fettsäurealkanolamide.

Beispiele für die in den erfindungsgemäßen Haarbehandlungsmitteln verwendbaren kationischen Tenside sind insbesondere quartäre Ammoniumverbindungen. Bevorzugt sind Ammoniumhalogenide wie Alkyltrimethylammoniumchloride, Dialkyldimethylammonium-chloride und Trialkylmethylammoniumchloride, beispielsweise Cetyltrimethylammonium-chlorid, Stearyltrimethylammoniumchlorid, Distearyldimethylammoniumchlorid, Lauryl-dimethylammoniumchlorid, Lauryldimethylbenzylammoniumchlorid und Tricetylmethyl-ammoniumchlorid. Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxylamino-modifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt; diquaternäre Polydimethylsiloxane, Quaternium-80).

Alkylamidoamine, insbesondere Fettsäureamidoamine wie das unter der Bezeichnung Tego Amid^{®}S 18 erhältliche Stearylamidopropyldimethylamin, zeichnen sich neben einer guten konditionierenden Wirkung speziell durch ihre gute biologische Abbaubarkeit aus. Ebenfalls sehr gut biologisch abbaubar sind quaternäre Esterverbindungen, sogenannte "Esterquats", wie die unter dem Warenzeichen Stepantex^{®} vertriebenen Methylhydroxyalkyldialkoyloxyalkylammoniummethosulfate.

Ein Beispiel für ein als kationisches Tensid einsetzbares quaternäres Zuckerderivat stellt das Handelsprodukt Glucquat^{®}100 dar, gemäß CTFA-Nomenklatur ein "Lauryl Methyl Gluceth-10 Hydroxypropyl Dimonium Chloride".

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, - hydroxide oder -alkoholate als Katalysatoren verwendet werden. Die Verwendung von Produkten mit eingeengter Homologenverteilung kann bevorzugt sein.

Weitere Wirk-, Hilfs- und Zusatzstoffe sind beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- kationische Polymere wie quaternisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quaternierte Dimethylaminoethylmethacrylat-Vinylpyrrolidon-Copolymere, Vinylpyrrolidon-Imidazoliniummethochlorid-Copolymere und quaternierter Polyvinylalkohol,
- zwitterionische und amphotere Polymere wie beispielsweise Acrylamidopropyltrimethylammoniumchlorid/Acrylat-Copolymere und Octylacrylamid/Methylmethacrylat /tert.-Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäure-anhydrid-Copolymere und Acrylsäure/Ethylacrylat/N-tert.-Butylacrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, beispielsweise Methylcellulose, Hydroxyalkylcellulose und Carboxymethylcellulose, Stärke-Fraktionen und Derivate wie Amylose, Amylopektin und Dextrine, Tone wie beispielsweise Bentonit oder vollsynthetische Hydrokolloide wie beispielsweise Polyvinylalkohol,
- Strukturanten wie Glucose und Maleinsäure,
- haarkonditionierende Verbindungen wie Phospholipide, beispielsweise Sojalecithin, Ei-Lecitin und Kephaline, sowie Silikonöle,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsvermittler wie Ethanol, Isopropanol, Ethylenglykol, Propylenglykol, Glycerin und Diethylenglykol,
- Antischuppenwirkstoffe wie Piroctone Olamine und Zink Omadine,
- weitere Substanzen zur Einstellung des pH-Wertes,
- Wirkstoffe wie Panthenol, Pantothensäure, Allantoin, Pyrrolidoncarbonsäuren und deren Salze, Pflanzenextrakte und Vitamine,
- Cholesterin,
- Lichtschutzmittel,
- Konsistenzgeber wie Zuckerester, Polyolester oder Polyolalkylether,
- Fette und Wachse wie Walrat, Bienenwachs, Montanwachs, Paraffine, Fettalkohole und Fettsäureester,
- Fettsäurealkanolamide,
- Komplexbildner wie EDTA, NTA und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate, Imidazole, Tannine, Pyrrol,
- Trübungsmittel wie Latex,
- Perlglanzmittel wie Ethylenglykolmono- und -distearat,
- Treibmittel wie Propan-Butan-Gemische, N₂O, Dimethylether, CO₂ und Luft sowie
- Antioxidantien.

Die Bestandteile des wasserhaltigen Trägers werden zur Herstellung der erfindungsgemäßen Färbemittel in für diesen Zweck üblichen Mengen eingesetzt; beispielsweise werden Emulgiermittel in Konzentrationen von 0,5 bis 30 Gew.-% und Verdickungsmittel in Konzentrationen von 0,1 bis 25 Gew.-% des gesamten Färbemittels eingesetzt.

Für das Färbeergebnis kann es vorteilhaft sein, den Färbemitteln Ammonium- oder Metallsalze zuzugeben. Geeignete Metallsalze sind beispielsweise Formiate, Carbonate, Halogenide, Sulfate, Butyrate, Valeriate, Capronate, Acetate, Lactate, Glykolate, Tartrate, Citrate, Gluconate, Propionate, Phosphate und Phosphonate von Alkalimetallen, wie Kalium, Natrium oder Lithium, Erdalkalimetallen, wie Magnesium, Calcium, Strontium oder Barium, oder von Aluminium, Mangan, Eisen, Kobalt, Kupfer oder Zink, wobei Natriumacetat, Lithiumbromid, Calciumbromid, Calciumgluconat, Zinkchlorid, Zinksulfat, Magnesiumchlorid, Magnesiumsulfat, Ammoniumcarbonat, -chlorid und - acetat bevorzugt sind. Diese Salze sind vorzugsweise in einer Menge von 0,03 bis 65 mmol, insbesondere von 1 bis 40 mmol, bezogen auf 100 g des gesamten Färbemittels, enthalten. Der pH-Wert der gebrauchsfertigen Färbezubereitungen liegt üblicherweise zwischen 2 und 12, vorzugsweise zwischen 4 und 10.

Ein zweiter Gegenstand der vorliegenden Erfindung betrifft die Verwendung der erfindungsgemäßen Mittel zur Färbung keratinischer Fasern.

Ein dritter Gegenstand der vorliegenden Erfindung betrifft ein Verfahren zum Färben von keratinhaltigen Fasern, insbesondere menschlichen Haaren, worin ein erfindungsgemäßes Färbemittel auf die keratinhaltigen Fasern aufgebracht, einige Zeit, üblicherweise ca. 30 Minuten, auf der Faser belassen und anschließend wieder ausgespült oder mit einem Shampoo ausgewaschen wird.

Die Verbindungen der Formel 1) oder 2) und die gegebenenfalls enthaltenen Oxidationsfarbstoffvorprodukte, Farbverstärker und direktziehenden Farbstoffe können entweder gleichzeitig auf das Haar aufgebracht werden oder aber auch nacheinander, wobei es unerheblich ist, welche der Komponenten zuerst aufgetragen wird. Die fakultativ enthaltenen Ammonium- oder Metallsalze können dabei der ersten oder der zweiten Komponente zugesetzt werden. Zwischen dem Auftragen der ersten und der zweiten Komponente können bis zu 30 Minuten Zeitabstand liegen. Auch eine Vorbehandlung der Fasern mit der Salzlösung ist möglich.

Die Verbindungen der Formel 1) oder 2) und die gegebenenfalls enthaltenen Oxidationsfarbstoffvorprodukte, Farbverstärker und direktziehenden Farbstoffe können entweder getrennt oder zusammen gelagert werden, entweder in einer flüssigen bis pastösen Zubereitung (wäßrig oder wasserfrei) oder als trockenes Pulver. Bei der getrennten Lagerung werden die Komponenten erst unmittelbar vor der Anwendung innig miteinander vermischt. Bei der trockenen Lagerung wird vor der Anwendung üblicherweise eine definierte Menge warmen (30 bis 80°C) Wassers hinzugefügt und eine homogene Mischung hergestellt.

### Beispiele

### Zusammensetzung und Herstellung der Färbecreme

Die Färbecreme wurde mit den folgenden Komponenten und nach folgendem Herstellungsverfahren zubereitet (alle Angaben sind, soweit nicht anders vermerkt, in g):

| | |
|---|---|
| **A** Hydrenol^{®} D¹ | 6,0 |
| **B** Lorol^{®} techn.² | 2,0 |
| **C** Cremophor^{®} A25³ | 2,0 |
| **D** Wasser | 60,0 |
| **E** (NH₄)₂SO₄ | 1,0 |
| **F** Farbstoff / Farbstoffgemische | 0,3 |
| **G** Ammoniumhydroxidlösung (25%ig) | ad pH9 |
| **H** Wasser | ad 100g |

| | |
|---|---|
| ¹ Cetearyl Alcohol (Cognis) ² C₁₂-C₁₈-Fettalkohol (Cognis) ³ Ceteareth-25 (BASF) | |

Die Substanzen A-C wurden bei 80°C aufgeschmolzen, mit 80°C heißem Wasser (D) vermischt und unter starkem Rühren emulgiert. Anschließend wurde die Emulsion unter schwachem Rühren abgekühlt. Die Farbstoffe (F) wurden in 50°C heißem Wasser (H) unter Zugabe von Ammoniumsulfat (E) und Ammoniak (G) auf pH9 eingestellt und mit Wasser auf 100 g aufgefüllt. Es wurde bis zum Erreichen der Raumtemperatur weitergerührt.

Die Färbecreme wurde auf ca. 5 cm lange Strähnen standardisierten, zu 90% ergrauten, aber nicht besonders vorbehandelten Menschenhaares aufgetragen und dort 30 Minuten bei 32°C belassen. Nach Beendigung des Färbeprozesses wurde das Haar gespült, mit einem üblichen Haarwaschmittel gewaschen und anschließend getrockent.

Die verwendeten Färbekomponenten sowie die Färbeergebnisse sind in der folgenden Tabelle dargestellt:

| **Verbindung** | **Farbton** |
|---|---|
| 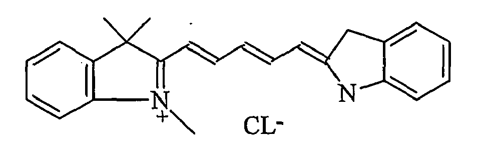 | Prinzeßblau |
| 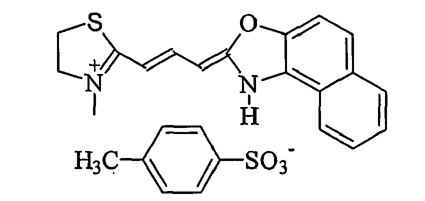 | Orange |
| 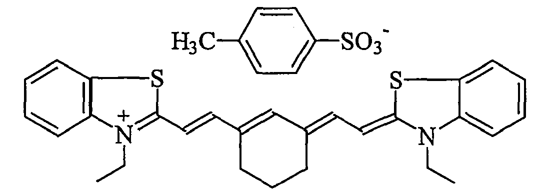 | Türkis |
| 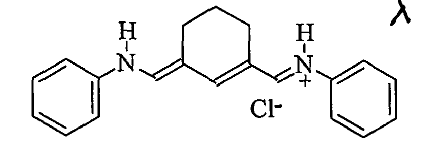 (nicht erfindungsgemäß) | Dottergelb |
| 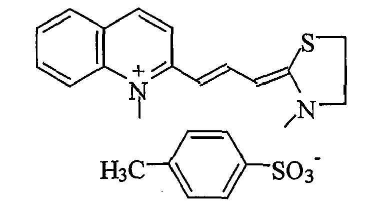 | Weinrot |
| 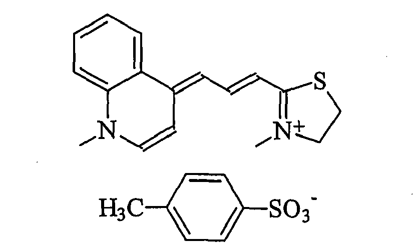 | Dunkelviolett |

## Patentansprüche

1. Mittel zum Färben von keratinhaltigen Fasern, insbesondere von menschlichen Haaren, enthaltend als färbende Komponente Verbindungen der Formel
1) A4 oder
2) A=B=A',
in denen =B= steht für eine durchgehende Doppelbindung zwischen den Gruppierungen A und A', oder =B= steht für die Gruppen B1 bis B3, 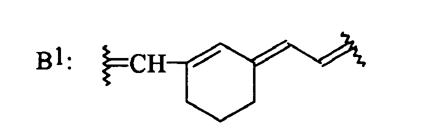 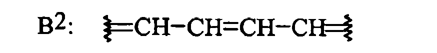 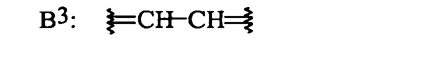 A ist ausgesucht aus den Gruppen A1 bis A3, 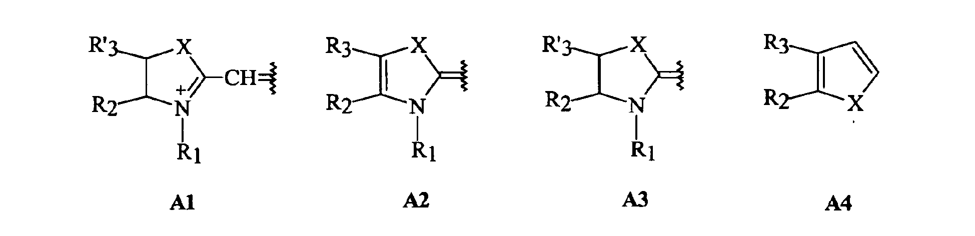 A' ist ausgewählt aus den Gruppen A11 bis A14, 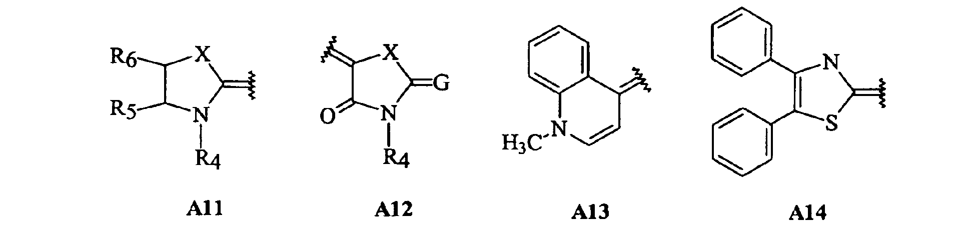 wobei
X steht für ein Schwefelatom, ein Sauerstoffatom, eine Gruppe NR', CR'R" oder eine Gruppe gemäß einer der Formeln X1 bis X4, 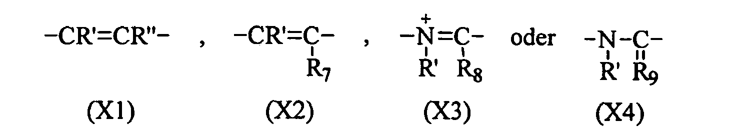 wobei R' und R" unabhängig voneinander stehen für Wasserstoff oder eine C₁-C₄-Alkylgruppe, die gegebenfalls mit einer SO₃ -Gruppe substituiert ist, R₇ steht für einen Rest gemäß der Formel (I), 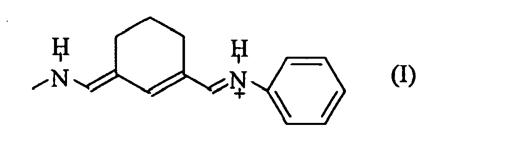 R₈ steht für einen Rest gemäß der Formel (II) 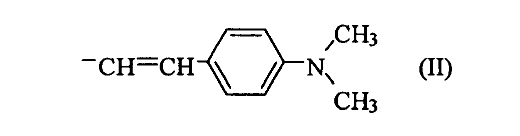 und R₉ steht für einen Rest gemäß Formel A12,
R₁ und R₄ stehen unabhängig voneinander für Wasserstoff, eine Aminogruppe, eine C₁-C₄-Alkylgruppe, die gegebenfalls mit einer oder mehreren Hydroxygruppe(n) oder einer Säure- oder Säureaniongruppe, insbesondere einer SO₃ ⁻Gruppe, substituiert sein kann, für eine C₁-C₄-Alkenylgruppe sowie für eine Formyl-, Acetyl-, Propionyl- und Buturylamidogruppe,
R₂ und R₃, R₂ und R'₃ sowie R₅ und R₆ können unabhängig voneinander stehen für Wasserstoff, eine C₁-C₄-Alkylgruppe, eine Formyl- oder Acetyl- C₁-C₄-alkylestergruppe, eine Phenylgruppe oder sie können jeweils gemeinsam eine orthoankondensierte Cyclohexyl-, Benzyl- oder eine Naphthylgruppe bilden, die gegebenenfalls mit einem oder mehreren der folgenden Gruppen substituiert sein können: C₁-C₄-Alkyl, C₂-C₄-Mono- oder Polyhydroxyalkyl, Halogen, C₁-C₄-Alkoxy sowie Formyl- oder Acetyl- C₁-C₄-alkylester; des weiteren kann R'₃ auch durch eine Doppelbindung an den Ring gebunden sein und für die Formel (III) stehen. 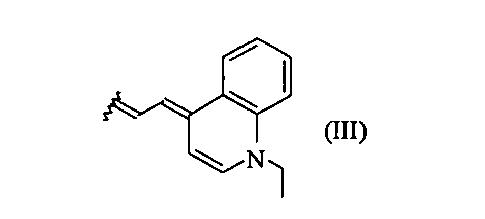 und G steht für ein Schwefelatom oder für eine Gruppe gemäß Formel (IV), 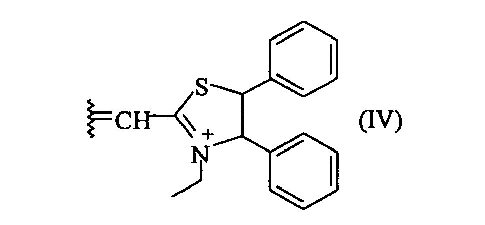 wobei diejenigen Formeln, die eine positive Ladung tragen weiterhin noch ein Gegenion enthalten, welches ausgesucht ist aus der Gruppe Halogenid, Ethylsulfat und 4-Methyl-benzol-sulfonat, **dadurch gekennzeichnet, dass** als Verbindungen mit der Formel A4 oder A=B=A' eine der in der Tabelle 1 genannten Verbindungen eingesetzt wird, wobei die in den Tabellen nicht definierten Substituenten jeweils für Wasserstoff stehen:
**Tabelle1**
| | **A** | **R₁** | **R₂/R₃ bzw. R₁/R'₃** | **B** | **A'** | **R₄** | **R₅/R₆** | **G** | **Gegenion** |
|---|---|---|---|---|---|---|---|---|---|
| **Verbindung 1** | A1 mit X = CR'R" Mit R' = R" = Methyl | Methyl | Benzol (ortho-ankondensiert) | B2 | A11 mit X = CR'R" mit R' = R" = Wasserstoff | Wasserstoff, | Benzol (ortho-ankondensiert) | - | Chlorid |
| **Verbindung 2** | A1 mit X = S | Methyl | R₂=R'₃= Wasserstoff | B3 | A11 mit X = O | Wasserstoff | Naphthalin (ortho-ankondensiert) | - | ₄-Methyl-benzolsulfonat |
| **Verbindung 3** | A1 mit X = S | Ethyl | Benzol (ortho-ankondensiert) | B1 | A11 mit X = S | Ethyl | Benzol (ortho-ankondensiert) | - | 4-Methyl-benzolsulfonat |
| **Verbindung 4** | A4 mit X = X2 mit R' = Wasserstoff und R₇ = Formel (I) | - | - | - | - | - | - | - | Chlorid |
| **Verbindung 5** | A1 mit X = X1 mit R'=R"= Wasserstoff | Methyl | Benzol (ortho-ankondensiert) | B3 | A11 mit X = S | Methyl | R₅ = R₆ = Wasserstoff | - | 4-Methyl-benzolsulfonat |
| **Verbindung 6** | A1 mit X = S | Methyl | R₂ = R'₃ = Wasserstoff | B3 | A13 | - | - | - | 4-Methyl-benzolsulfonat |
| **Verbindung 7** | A1 mit X = S | Allyl | R₂ = Wasserstoff R'₃ = Formel (III) | Durchgehende Doppelbindung | A14 | - | - | - | Ethylsulfat |
| **Verbindung 8** | A3 mit X = S | Methyl | R₂ = R₃ = Wasserstoff | B3 | A12 mit X = O | Ethyl | - | S | - |
| **Verbindung 9** | A3 mit X = CR'R" Mit R' = R" = Wasserstoff | Methyl | R₂ = R'₃ = Wasserstoff | B3 | A12 mit X = NR' mit R' = Methyl | Methyl | - | S | - |
| **Verbindung 10** | A4 mit X = X3 mit R' = Ethyl R₈ = Formel (II) | - | - | - | - | - | - | - | Iodid |
| **Verbindung 11** | A2 mit X = S | Ethyl | R₂ = R₃ Methyl | Durchgehende Doppelbindun g | A12 mit X = S | Amino | - | S | - |
| **Verbindung 12** | A3 mit X = S | Ethyl | Benzol (ortho-ankondensiert) | B3 | A12 mit X = S | Carboxy-methyl | - | S | - |
| **Verbindung 13** | A3 mit X = S | Ethyl | R₂ = R'₃ = Phenyl | B3 | A12 mit X = S | Allyl | - | Formel (IV) | Ethylsulfat |
| **Verbindung 14** | A3 mit X = CR'R" mit R' = R" = Wasserstoff | Methyl | R₂ = R'₃ = Wasserstoff | B3 | A12 mit X = O | Ethyl | - | S | - |
| **Verbindung 15** | A4 mit X = X₄ mit R' = Methyl und R₉=A12; mit X= S,G=S,R₄= Methyl | - | - | - | - | - | - | - | - |
| **Verbindung 16** | A4 mit X = X₄ mit R' = Methyl und R₉ = A12; mit X = O,G=S,R₄= Methyl | - | - | - | - | - | - | - | - |
| **Verbindung 17** | A2 mit X = S | Ethyl | R₂ = R₃ Methyl | Durchgehende Doppelbindung | A12 mit X = S | Acetamido | - | S | - |
| **Verbindung 18** | A3 mit X = S | Methyl | R₂ = R'₃ Wasserstoff | B3 | A12 mit X = NR' mit R' = Methyl | Wasserstoff | - | S | - |
| **Verbindung 19** | A3 mit X = O | Carboxy-ethyl | Benzol (ortho-ankondensiert) | B3 | A12mit X = NR' mit R' = Propyl | 2-Hydroxyeth yl | - | S | - |
| **Verbindung 20** | A3 mit X = S | Methyl | R₂ = R'₃ = Wasserstoff | B3 | A12 mit X = S | Allyl | - | S | - |
| **Verbindung 21** | A3 mit X = CR'R" mit R' = R" = Wasserstoff | Methyl | R₂ = R'₃ = Wasserstoff | B3 | A12 mit X = S | Allyl | - | S | - |
| **Verbindung 22** | A2 mit X = S | Ethyl | R₂ = R₃ Methyl | Durchgehend e Doppelbindung | A12 mit X = S | Allyl | - | S | - |
| **Verbindung 23** | A2 mit X = S | Ethyl | R₂ = Wasserstoff R₃ = Phenyl | Durchgehend e Doppelbindung | A12 mit X = S | Allyl | - | S | - |
| **Verbindung 24** | A2 mit X = S | Methyl | R₂ = Ethylcarboxymethyl R₃ = Wasserstoff | Durchgehend e Doppelbindung | A12 mit X = S | Allyl | - | S | - |
| **Verbindung 25** | A3 mit X = O | Carboxy -ethyl | Benzol (ortho-ankondensiert) | B3 | A12 mit X = NR' mit R' = Methyl | Methyl | - | S | - |
| **Verbindung 26** | A3 mit X = NR' mit R' = Ethyl | Ethyl | Ethylbenzoat (arom. Substituent nicht benachbart zur Kondensationsstelle) | B3 | A12 mit X = O | Ethyl | - | S | - |
| **Verbindung 27** | A3 mit X = NR' mit R' = Ethyl | Ethyl | Ethylbenzoat (arom. Substituent nicht benachbart zur Kondensationsstelle) | B3 | A12 mit X = S | Ethyl | - | S | - |

2. Mittel nach Anspruch 1, **dadurch gekennzeichnet, daß** es Farbverstärker ausgewählt aus der Gruppe bestehend aus Piperidin, Piperidin-2-carbonsäure, Piperidin-3-carbonsäure, Piperidin-4-carbonsäure, Pyridin, 2-Hydroxypyridin, 3-Hydroxypyridin, 4-Hydroxypyridin, Imidazol, 1-Methylimidazol, Histidin, Pyrrolidin, Prolin, Pyrrolidon, Pyrrolidon-5-carbonsäure, Pyrazol, 1,2,4-Triazol, Piperazin oder deren beliebigen Gemischen enthält.

3. Mittel nach einem der Ansprüche 1 bis 2, **dadurch gekennzeichnet, daß** es direktziehende Farbstoffe aus der Gruppe der Nitrophenylendiamine, Nitroaminophenole, Anthrachinone oder Indophenole vorzugsweise in einer Menge von 0,01 bis 20 Gew.-%, bezogen auf das gesamte Färbemittel, enthält.

4. Mittel nach einem der Ansprüche 1 bis 3, **dadurch gekennzeichnet, daß** Ammonium- oder Metallsalze ausgewählt aus der Gruppe der Formiate, Carbonate, Halogenide, Sulfate, Butyrate, Valeriate, Capronate, Acetate, Lactate, Glycolate, Tartrate, Citrate, Gluconate, Propionate, Phosphate und Phosphonate von Alkalimetallen, wie Kalium, Natrium oder Lithium, Erdalkalimetallen, wie Magnesium, Calcium, Strontium oder Barium, oder von Aluminium, Mangan, Eisen, Kobalt, Kupfer oder Zink, zugegeben werden.

5. Mittel nach einem der Ansprüche 1 bis 4, **dadurch gekennzeichnet, dass** es Oxidationsmittel, beispielsweise H₂O₂ oder Gemische von H₂O₂ und Peroxysalzen, beispielsweise Peroxydisulfit, enthält.

6. Mittel nach Anspruch 5, **dadurch gekennzeichnet, dass** es als Oxidationsmittel H₂O₂ in einer Menge von 0,01 - 6 Gew.-%, bezogen auf die Anwendungslösung, enthält.

7. Mittel nach einem der Ansprüche 1 bis 6, **dadurch gekennzeichnet, dass** es anionische, zwitterionische oder nichtionische Tenside enthält.

8. Verwendung von Mitteln gemäß eines der Ansprüche 1 bis 7 zum Färben von keratinhaltigen Fasern.

9. Verfahren zum Färben keratinhaltiger Fasern, insbesondere menschlichen Haaren, worin ein Färbemittel gemäß eines der Ansprüche 1 bis 7 auf die keratinhaltigen Fasern aufgebracht, einige Zeit, üblicherweise ca. 30 Minuten, auf der Faser belassen und anschließend wieder ausgespült oder mit einem Shampoo ausgewaschen wird.

## Claims

1. An agent for colouring keratin-containing fibres, in particular human hair, containing as the colouring component compounds of the formula
1) A4 or
2) A=B=A',
in which =B= denotes a continuous double bond between the groupings A and A', or =B= denotes groups B1 to B3, 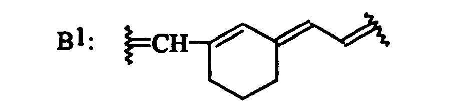 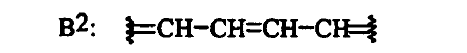 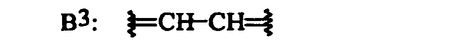
A is selected from groups A1 to A3, 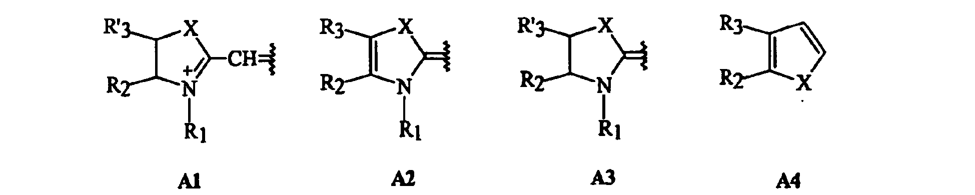
A' is selected from groups A11 to A14, 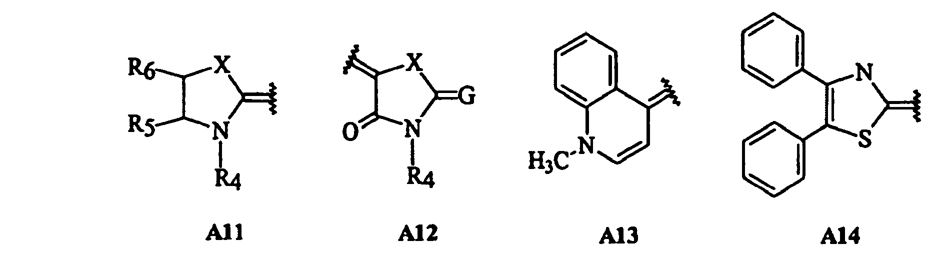
wherein
X denotes a sulfur atom, an oxygen atom, a group NR', CR'R" or a group of one of the formulae X1 to X4, 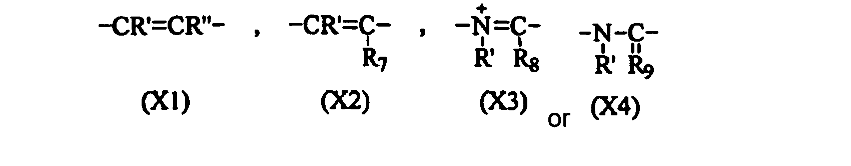 wherein R' and R" mutually independently denote hydrogen or a C₁-C₄ alkyl group which is optionally substituted with an SO₃⁻ group, R₇ denotes a residue of the formula (I), 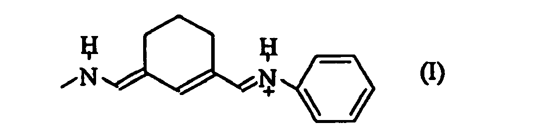
R₈ denotes a residue of the formula (II) 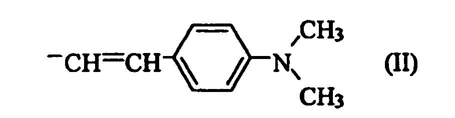
R₁ and and R₉ denotes a residue of the formula A12, R₄ mutually independently denote hydrogen, an amino group, a C₁-C₄ alkyl group, which may optionally be substituted with one or more hydroxyl group(s) or an acid or acid anion group, in particular an SO₃⁻ group, a C₁-C₄ alkenyl group and a formyl, acetyl, propionyl and butyrylamido group,
R₂ and R₃, R₂ and R'₃ and R₅ and R₆ may mutually independently denote hydrogen, a C₁-C₄ alkyl group, a formyl or acetyl C₁-C₄ alkyl ester group, a phenyl group or they may in each case together form an ortho-fused cyclohexyl, benzyl or naphthyl group, which may optionally be substituted with one or more of the following groups: C₁-C₄ alkyl, C₂-C₄ mono- or polyhydroxyalkyl residue, halogen, C₁-C₄ alkoxy and formyl or acetyl C₁-C₄ alkyl ester; R'₃ may moreover also be attached to the ring by a double bond and denote the formula (III), 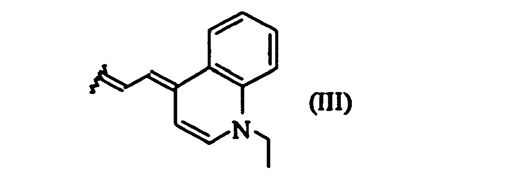
and G denotes a sulfur atom or a group of the formula (IV), 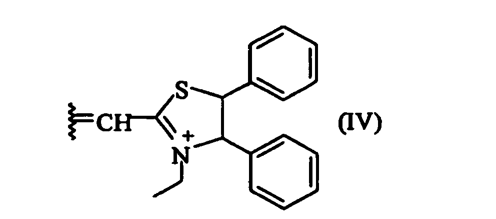 wherein those formulae which bear a positive charge furthermore also contain a counterion which is selected from the group halide, ethyl sulfate and 4-methylbenzene sulfonate, **characterised in that** the compounds of the formula A4 or A=B=A' used comprise one of the compounds stated in Table 1, wherein the substituents not defined in the table in each case denote hydrogen:
**Table 1 Table 1**
| | **A** | **R₁** | **R₂/R₃ or R₂/R'₃** | **B** | **A'** | **R₄** | **R₅/R₆** | **G** | **Counterion** |
|---|---|---|---|---|---|---|---|---|---|
| **Compound 1** | A1 with X = CR'R" with R' = R" = methyl | Methyl | Benzene (ortho-fussed) | B2 | A11 with X = CR'R" with R' = R" = hydrogen | Hydrogen | Benzene (ortho-fused) | - | Chloride |
| **Compound 2** | A1 with X = S | Methyl | R₂ = R'₃ hydrogen | = B3 | A11 with X = O Hydrogen | | Naphthalene -(ortho-fused) | - | 4-Methylbenzene sulfonate |
| **Compound 3** | A1 with X = S | Ethyl | Benzene (ortho-fused) | B1 | A11 with X = S | Ethyl | Benzene (ortho-fused) | - | 4-Methylbenzene sulfonate |
| **Compound 4** | A4 with X = X2 with R' = hydrogen and - R₇ = formula (I) | - | - | - | - | - | - | - | Chloride |
| **Compound 5** | A1 with X = X1 R' = R" = hydrogen | with Methyl | Benzene (ortho-fused) | B3 | A11 with X = S | Methyl | R₅ = R₆ = hydrogen | - | 4-Methylbenzene sulfonate |
| **Compound 6** | A1 with X = S | Methyl | R₂ = R'₃ hydrogen | B3 | A13 - | - | - | - | 4-Methylbenzene sulfonate |
| **Compound 7** | A1 with X = S | Allyl | R₂ = hydrogen = formula (III) | R'₃ Continuous double bond | A14 | - | - | - | Ethyl sulfate |
| **Compound 8** | A3 with X = S | Methyl | R₂ = R₃ hydrogen | - B3 | A12 with X = O | Ethyl | - | S | - |
| **Compound 9** | A3 with X = CR'R" with R' = R" = hydrogen | Methyl | R₂ = R'₃ = hydrogen | B3 | A12 with X = NR' with R' = methyl | Methyl | - | S | - |
| **Compound 10** | A4 with X = X3 with R' = ethyl R₈ formula (II) | - | - | - | - | - | - | - | Iodide |
| **Compound 11** | A2 with X = S | Ethyl | R₂ = R₃ = methyl | Continuous double bond | A12 with X = S | Amino | - | S | - |
| **Compound 12** | A3 with X = S | Ethyl | Benzene (ortho-fused) | B3 | A12 with X = S | Carboxy-methyl | - | S | - |
| **Compound 13** | A3 with X = S | Ethyl | R₂ = R'₃ = phenyl | B3 | A12 with X = S | Allyl | - | Formula | Ethyl sulfate |
| **Compound 14** | A3 with X = CR'R" with R' = R" = hydrogen | Methyl | hydrogen | B3 | A12 with X = O | Ethyl | - | S | - |
| **Compound 15** | A4 with X = X4 with R' = methyl and R₉ = A12; with X = S, G = S, R₄ = methyl | - | - | - | - | - | - | - | - |
| **Compound 16** | A4 with X = X4 with R' = methyl and R₉ = A12; with X = O, G = S, R₄ = methyl | - | - | - | - | - | - | - | - |
| **Compound 17** | A2 with X = S | Ethyl | R₂ = R₃ = methyl | Continuous double bond | A12 with X = S | Acetamido | - | S | - |
| **Compound 18** | A3 with X = S | Methyl | R₂ = R'₃ = hydrogen | B3 | A12 with X = NR' with R'= methyl | Hydrogen | - | S | - |
| **Compound 19** | A3 with X = O | Carb-oxyethyl | Benzene (ortho-fused) | B3 | A12 with X = NR' 2-Hydroxy- with R' = propyl | ethyl | - | S | - |
| **Compound 20** | A3 with X = S | Methyl | hydrogen R'₃ | B3 | A12 with X = S | Allyl | - | S | - |
| **Compound 21** | with R' = R" = hydrogen | Methyl | R₂ R'₃ = hydrogen | B3 | A12 with X = S | Allyl | - | S | - |
| **Compound 22** | A2 with X = S | Ethyl | R₂ = R₃ = methyl | Continuous double bond | A12 with X=S | Allyl | - | S | - |
| **Compound 23** | A2 with X = S | Ethyl | R₂ = hydrogen R₃ = phenyl | Continuous double bond | A12 with X = S A12 with X = S | Allyl | - | S | - |
| **Compound 24** | A2 with X = S | Methyl | R₂ = ethylcarboxymethyl R₃ | Continuous double bond | A12 with X = S | Allyl | - | S | - |
| **Compound 25** | A3 with X = O | Carboxyethyl | hydrogen Benzene (ortho-fused) | B3 | A12 with X = NR' with R' = methyl | Methyl | - | S | - |
| **Compound 26** | A3 with X = NR' with R' = ethyl | Ethyl | Ethyl benzoate (aromatic substituent not adjacent to site of fusion) | B3 | A12 with X = O | Ethyl | - | S | - |
| **Compound 27** | A3 with X = NR' with R' = ethyl | Ethyl | Ethyl benzoate (aromatic substituent not adjacent to site of fusion) | B3 | A12 with X = S | Ethyl | - | S | - |

2. An agent according to claim 1, **characterised in that** it contains colour enhancers selected from the group consisting of piperidine, piperidine-2-carboxylic acid, piperidine-3-carboxylic acid, piperidine-4-carboxylic acid, pyridine, 2-hydroxypyridine, 3-hydroxypyridine, 4-hydroxypyridine, imidazole, 1-methylimidazole, histidine, pyrrolidine, proline, pyrrolidone, pyrrolidone-5-carboxylic acid, pyrazole, 1,2,4-triazole, piperazine or any desired mixtures thereof.

3. An agent according to any one of claims 1 to 2, **characterised in that** it contains direct dyes from the group comprising nitrophenylenediamines, nitroaminophenols, anthraquinones or indophenols preferably in a quantity of 0.01 to 20 wt.% relative to the total colouring agent.

4. An agent according to any one of claims 1 to 3, **characterised in that** ammonium or metal salts are added which are selected from the group comprising formates, carbonates, halides, sulfates, butyrates, valerates, caproates, acetates, lactates, glycolates, tartrates, citrates, gluconates, propionates, phosphates and phosphonates of alkali metals, such as potassium, sodium or lithium, alkaline earth metals, such as magnesium, calcium, strontium or barium, or of aluminium, manganese, iron, cobalt, copper or zinc.

5. An agent according to any one of claims 1 to 4, **characterised in that** it contains oxidising agent, for example H₂O₂ or mixtures of H₂O₂ and peroxy salts, for example peroxydisulfite.

6. An agent according to claim 5, **characterised in that**, as oxidising agent, it contains H₂O₂ in a quantity of 0.01-6 wt.%, relative to the application solution.

7. An agent according to any one of claims 1 to 6, **characterised in that** it contains anionic, zwitterionic or nonionic surfactants.

8. Use of agents according to any one of claims 1 to 7 for colouring keratin-containing fibres.

9. A method of colouring keratin-containing fibres, in particular human hair, in which a colouring agent according to any one of claims 1 to 7 is applied onto the keratin-containing fibres, left on the fibres for a period of time, conventionally approx. 30 minutes, and then rinsed out again or washed out with a shampoo.

## Revendications

1. Agent pour la teinture de fibres kératiniques, en particulier de cheveux humains, contenant, à titre de composant de teinture, des composés répondant à la formule
1) A4 ou
2) A=B=A'
dans lesquelles =B= représente une liaison double continue entre les groupements A et A' ou bien =B= représente les groupes B1 à B3 : 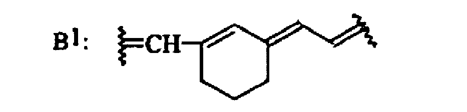 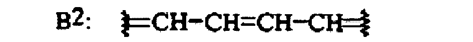 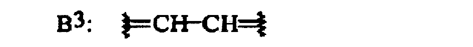 A est choisi parmi les groupes A1 à A3 : 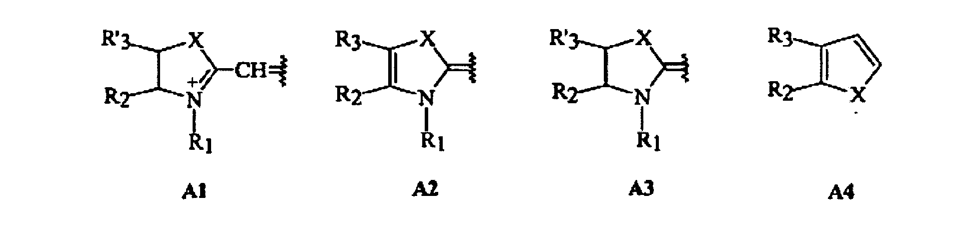 A' est choisi parmi les groupes A11 à A14 : 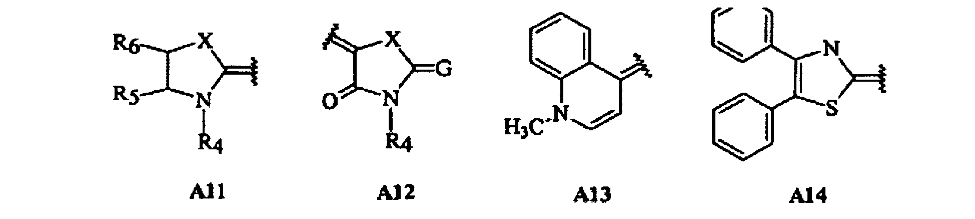 dans lesquels
X représente un atome de soufre, un atome d'oxygène, un groupe NR',
un groupe CR'R" ou un groupe répondant à une des formules X1 à X4 : 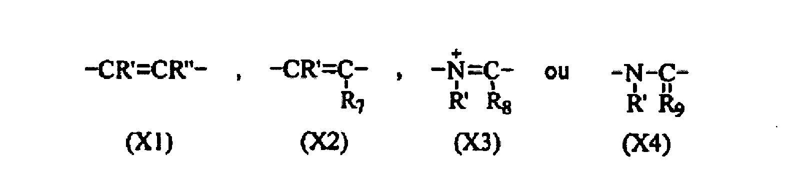 dans lesquelles R' et R" représentent, indépendamment l'un de l'autre, un atome d'hydrogène ou un groupe alkyle en C₁-C₄, qui est éventuellement substitué avec un groupe SO₃⁻ ;
R₇ représente un radical répondant à la formule (I) : 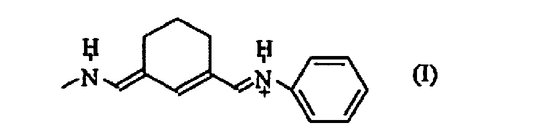 R₈ représente un radical répondant à la formule (II) : 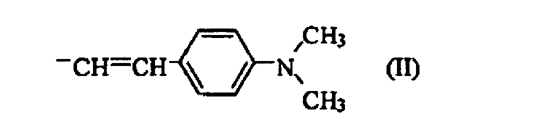 et
R₉ représente un radical répondant à la formule A12 ;
R₁ et R₄ représentent, indépendamment l'un de l'autre, un atome d'hydrogène, un groupe amino, un groupe alkyle en C₁-C₄, qui peut être éventuellement substitué avec un ou plusieurs groupes hydroxyle ou bien avec un groupe acide ou un groupe anionique d'acide, en particulier un groupe SO₃⁻, un groupe alcényle en C₁-C₄, ainsi qu'un groupe formyle, un groupe acétyle, un groupe propionyle et un groupe butyrylamido ;
R₂ et R₃, R₂ et R'₃ et R₅ et R₆ peuvent représenter, indépendamment l'un de l'autre, un atome d'hydrogène, un groupe alkyle en C₁-C₄, un groupe formyl- ou acétyl-ester alkylique en C₁-C₄, un groupe phényle ; ou bien, peuvent former respectivement ensemble un groupe cyclohexyle, un groupe benzyle ou un groupe naphtyle orthocondensés qui peuvent être éventuellement substitués avec un ou plusieurs groupes parmi les groupes suivants : un groupe alkyle en C₁-C₄, un groupe mono- ou polyhydroxyalkyle en C₂-C₄, un atome d'halogène, un groupe alcoxy en C₁-C₄, et un groupe formyl- ou acétyl-ester alkylique en C₁-C₄; en outre,
R'₃ peut également être lié au noyau via une liaison double et répondre à la formule (III). 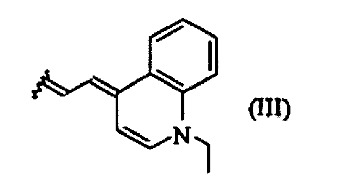 et G représente un atome de soufre ou bien un groupe répondant à la formule (IV) : 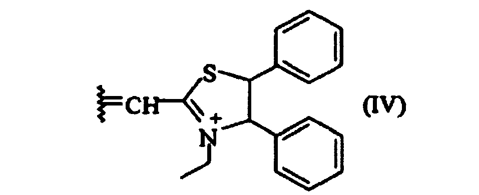 les formules qui portent une charge positive contenant en outre un contre-ion qui est choisi parmi le groupe comprenant un halogénure, un sulfate d'éthyle et un 4-méthyl-benzènesulfonate, **caractérisé en ce qu'**on met en oeuvre, à titre de composés répondant à la formule A4 ou à la formule A=B=A', un des composés mentionnés dans le tableau 1, les substituants non définis dans les tableaux représentant respectivement un atome d'hydrogène :
**tableau 1 Tableau 1**
| | A | R₁ | R₁/R₃, resp. R₂/R'₃ | B | A' | R₄ | R₅/R₆ | G | Contre-ion |
|---|---|---|---|---|---|---|---|---|---|
| Composé 1 | A1 avec X = CR'R" avec R' = R" = méthyle | méthyle | Benzène (condensé en ortho) | B2 | A11 avec X = CR'R" avec R' = R" = hydrogène | Hydrogène | Benzène (condensé en ortho) | - | Chlorure |
| Composé 2 | A1 avec X = S | méthyle | R₂ = R'₃ = hydrogène | B3 | A11 avec X = O | Hydrogène | Naphtalène (condensé en ortho) | - | 4-méthyl-benzène-sulfonate |
| Composé 3 | A1 avec X = S | éthyle | Benzène (condensé en ortho) | B1 | A11 avec X = S | Éthyle | Benzène (condensé en ortho) | - | 4-méthyl-benzène-sulfonate |
| Composé 4 | A4 avec X = X2 avec R' = hydrogène et R₇ = formule (I) | - | - | - | - | - | - | - | Chlorure |
| Composé 5 | A1 avec X = X1 avec R' = R" = hydrogène | méthyle | Benzène (condensé en ortho) | B3 | A11 avec X = S | Méthyle | R₅ = R₆ = hydrogène | - | 4-méthyl-benzène-sulfonate |
| Composé 6 | A1 avec X = S | méthyle | R₂ = R'₃ = hydrogène | B3 | A13 | - | - | - | 4-méthyl-benzène-sulfonate |
| Composé 7 | A1 avec X = S | allyle | R₂ = hydrogène R'₃ = formule (III) | Liaison double continue | A14 | - | - | - | Sulfate d'éthyle |
| Composé 8 | A3 avec X = S | Méthyle | R₂ = R₃ = hydrogène | B3 | A12 avec X = O | Éthyle | - | S | - |
| Composé 9 | A3 avec X = CR'R" avec R' = R" = hydrogène | Méthyle | R₂ = R'₃ = hydrogène | B3 | A12 avec X = NR' avec R' = méthyle | Méthyle | - | S | - |
| Composé 10 | A4 avec X = X3 avec R'= éthyle ; R₈ = formule (II) | - | - | - | - | - | - | - | Iodure |
| Composé 11 | A2 avec X = S | Éthyle | R₂ = R₃ = méthyle | Liaison double continue | A12 avec X = S | Amino | - | S | - |
| Composé 12 | A3 avec X = S | Éthyle | Benzène (condensé en ortho) | B3 | A12 avec X = S | Carboxy-méthyle | - | S | - |
| Composé 13 | A3 avec X = S | Éthyle | R₂ = R'₃ = phényle | B3 | A12 avec X = S | Allyle | - | Formule (IV) | Sulfate d'éthyle |
| Composé 14 | A3 avec X = CR'R" avec R' = R" = hydrogène | Méthyle | R₂ = R'₃ = hydrogène | B3 | A12 avec X = O | Éthyle | - | S | - |
| Composé 15 | A4 avec X = X4 avec R'= méthyle et R₉ = A12 ; avec X = S, G = S, R₄ = méthyle | - | - | - | - | - | - | - | - |
| Composé 16 | A4 avec X = X4 avec R' = méthyle et R₉ = A12; avec X= O, G = S, R₄ = méthyle | - | - | - | - | - | - | - | - |
| Composé 17 | A2 avec X = S | Éthyle | R₂ = R₃ = méthyle | Liaison double continue | A12 avec X = S | Acétamido | - | S | - |
| Composé 18 | A3 avec X = S | Méthyle | R₂ = R'₃ = hydrogène | B3 | A12 avec X = NR' avec R' = méthyle | Hydrogène | - | S | - |
| Composé 19 | A3 avec X = O | Carboxy-éthyle | Benzène (condensé en ortho) | B3 | A12 avec X = NR' avec R' = | 2-hydroxyéthyle | - | S | - |
| Composé 20 | A3 avec X = S | Méthyle | R₂ = R'₃ = hydrogène | B3 | propyle A12 avec X = S | Allyle | - | S | - |
| Composé 21 | A3 avec X = CR'R" avec R' = R" = hydrogène | Méthyle | R₂ = R'₃ = hydrogène | B3 | A12 avec X = S | Allyle | - | S | - |
| Composé 22 | A2 avec X = S | Éthyle | R₂ = R₃ = méthyle | Liaison double continue | A12 avec X = S | Allyle | - | S | - |
| Composé 23 | A2 avec X = S | Éthyle | R₂ = hydrogène R₃ = phényle | Liaison double continue | A12 avec X = S | Allyle | - | S | - |
| Composé 24 | A2 avec X = S | Méthyle | R₂ = éthyl-carboxyméthyle R₃ = hydrogène | Liaison double continue | A12 avec X = S | Allyle | - | S | - |
| Composé 25 | A3 avec X = O | Carboxy-éthyle | Benzène (condensé en ortho) | B3 | A12 avec X = NR' avec R' = méthyle | Méthyle | - | S | - |
| Composé 26 | A3 avec X = NR' avec R' = éthyle | Éthyle | Benzoate d'éthyle (substituant aromatique non voisin de l'endroit de condensation) | B3 | A12 avec X = O | Éthyle | - | S | - |
| Composé 27 | A3 avec X = NR' avec R' = éthyle | Éthyle | Benzoate d'éthyle (substituant aromatique non voisin de l'endroit de condensation) | B3 | A12 avec X = S | Éthyle | - | S | - |
| | | | | | | | | | |

2. Agent selon la revendication 1, **caractérisé en ce qu'**il contient des renforçateurs des couleurs choisis parmi le groupe constitué par la pipéridine, l'acide pipéridine-2-carboxylique, l'acide pipéridine-3-carboxylique, l'acide pipéridine-4-carboxylique, la pyridine, la 2-hydroxypyridine, la 3-hydroxypyridine, la 4-hydroxypyridine, l'imidazole, le 1-méthylimidazole, l'histidine, la pyrrolidine, la proline, la pyrrolidone, l'acide pyrrolidone-5-carboxylique, le pyrazole, le 1,2,4-triazole, la pipérazine ou l'un quelconque de leurs mélanges.

3. Agent selon l'une quelconque des revendications 1 à 2, **caractérisé en ce qu'**il contient des colorants directs du groupe des nitrophénylènediamines, des nitroaminophénols, des anthraquinones ou des indophénols, de préférence en une quantité de 0,01 à 20 % en poids, rapportés à l'agent de teinture dans sa totalité.

4. Agent selon l'une quelconque des revendications 1 à 3, **caractérisé en ce qu'**on ajoute des sels d'ammonium ou des sels métalliques choisis parmi le groupe des formiates, des carbonates, des halogénures, des sulfates, des butyrates, des valérates, des caproates, des acétates, des lactates, des glycolates, des tartrates, des citrates, des gluconates, des propionates, des phosphates et des phosphonates de métaux alcalins tels que le potassium, le sodium ou le lithium, de métaux alcalino-terreux tels que le magnésium, le calcium, le strontium ou le baryum, ou encore de l'aluminium, du manganèse, du fer, du cobalt, du cuivre ou du zinc.

5. Agent selon l'une quelconque des revendications 1 à 4, **caractérisé en ce qu'**il contient des agents d'oxydation, par exemple du H₂O₂, ou des mélanges de H₂O₂ et de peroxysels, par exemple le peroxydisulfite.

6. Agent selon la revendication 5, **caractérisé en ce qu'**il contient, à titre d'agent d'oxydation, du H₂O₂, en une quantité de 0,01 à 6 % en poids, rapportés à la solution d'utilisation.

7. Agent selon l'une quelconque des revendications 1 à 6, **caractérisé en ce qu'**il contient des agents tensioactifs anioniques, zwitterioniques ou non ioniques.

8. Utilisation d'agents selon l'une quelconque des revendications 1 à 7, pour la teinture de fibres kératiniques.

9. Procédé pour la teinture de fibres kératiniques, en particulier de cheveux humains, dans lequel on applique un agent de teinture selon l'une quelconque des revendications 1 à 7 sur les fibres kératiniques, on le maintient pendant un certain temps, habituellement pendant environ 30 minutes, sur les fibres, et ensuite on l'en retire par rinçage ou bien par lavage avec un shampooing.
